Europäisches Patentamt

European Patent Office

Office européen des brevets

(11) Publication number: **0 227 400 B1**

(19)

(12) **EUROPEAN PATENT SPECIFICATION**

(45) Date of publication of patent specification: **04.12.91**   (51) Int. Cl.⁵: **A61K 37/54, A61K 9/50**

(21) Application number: **86309754.9**

(22) Date of filing: **15.12.86**

(54) Inhibition of post-surgical adhesion formation by the topical administration of tissue plasminogen activator.

(30) Priority: **16.12.85 US 809536**

(43) Date of publication of application:
**01.07.87 Bulletin 87/27**

(45) Publication of the grant of the patent:
**04.12.91 Bulletin 91/49**

(84) Designated Contracting States:
**BE CH DE FR GB GR IT LI NL SE**

(56) References cited:
**EP-A- 0 041 449**        **EP-A- 0 143 949**
**EP-A- 0 174 835**        **EP-A- 0 219 076**
**FR-A- 2 126 270**        **GB-A- 2 042 556**
**GB-A- 2 119 804**

**CHEMICAL ABSTRACTS, vol. 97, no. 22, 29th
November 1982, page 376, no. 188156k, Co-
lumbus, Ohio, US; M. MEZEI: "Liposomes, a
selective drug delivery system for the topi-
cal route of administration: gel dosage
form" & J. PHARM. PHARMACOL. 1982, 34(7),
473-4**

**CHEMICAL ABSTRACTS, vol. 83, no. 5, 4th
August 1975, page 42, no. 37708b, Columbus,
Ohio, US; A.B. GAZZANIGA et al.:**

**"Prevention of peritoneal adhesions in the
rat. Effects of dexamethasone, methylpred-
nisolone, promethazine, and human
fibrinolysin" && ARCH. SURG. (CHICAGO)
1975, 110(4). 429-32**

(73) Proprietor: **ETHICON INC.**
**U.S. Route 22**
**Somerville New Jersey 08876(US)**

(72) Inventor: **Sheffield, Warren D.**
**RD 3 Box 67**
**Blossom Hill Road Lebanon, NJ(US)**
Inventor: **Dizerega, Gere D.**
**420 San Juan Place**
**Pasadena, CA(US)**

(74) Representative: **Jones, Alan John et al**
**CARPMAELS & RANSFORD 43 Bloomsbury
Square**
**London, WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to the inhibition of post-surgical adhesion formation.

Adhesion formation is a major post-surgical complication with no practical solution. The incidence of adhesion formation following surgery approaches 100 per cent, according to some sources, with a clinically significant complication rate of about 5 to 10 per cent, depending on the type of surgery. Among such complications are bowel obstruction, infertility, and pain. Occasionally, adhesions necessitate a second operative procedure to remove the adhesion, which may in turn further aggravate the problem.

Because of the seriousness of the problem, much medical research has been performed in efforts to find ways to combat adhesions. See, for instance, Stangel et al., "Formation and Prevention of Postoperative Abdominal Adhesions", the Journal of Reproductive Medicine, Vol. 29, No. 3, March 1984 (pages 143-156), and diZerega, "The Cause and Prevention of Postsurgical Adhesions", published by Pregnancy Research Branch, National Institute of Child Health and Human Development, National Institutes of Health, Building 18, Room 101, Bethesda, MD 20205.

Among the approaches that have been tried for preventing post-surgical adhesion are the following:

Systemic administration of ibuprofen (e.g., see Singer, U.S. Patent No. 4,346,108);

Parenteral administration of antihistamines, corticosteroids, and antibiotics;

Intraperitoneal administration of dextran solution and of polyvinylpyrrolidone solution; and

Systemic administration of oxyphenbutazone, a non-steroidal anti-inflammatory drug that acts by inhibiting prostaglandin production.

Corticosteroids have been administered intraperitoneally as well as systemically in efforts to prevent adhesions. (See the Stangel et al. article, cited above, on page 147, as well as the articles cited therein.) Some studies have questioned the efficacy of corticosteroids in adhesion prevention. In high doses, these materials may suppress the immune system and interfere with wound healing. Therefore, the use of corticosteroids does not seem to be an acceptable solution to the post-operative adhesion problem.

Kapur et al., in "Prevention of Reformation of Peritoneal Adhesions". Arch. Surg., Vol. 105, Nov. 1972 (pages 761-764), disclose the systemic administration of proteolytic enzymes from carica papaya to combat adhesions.

Singh, in U. S. Patent No. 3,912,704, suggests that a protease inhibitor isolated from horse urine may be useful in preventing the formation of post surgical adhesions.

The use of streptokinase, streptodornase, and urokinase in preventing adhesions has been reported. Administration was by a single intraperitoneal dose. The references are:

Ascherl et al., PREVENTION OF INTRAPERITONEAL ADHESIONS WITH A FIBRINOLYTIC, Medwelt, 34, No. 13/83, pp. 410-415;

Mund-Hoym et al., PREVENTION OF POSTOPERATIVE ADHESIONS-AN ANIMAL STUDY, Geburtsh, u. Franenheilk, 44 (1984), pp 463-467; and

Minju et al., ANIMAL STUDIES ON THE PREVENTION OF ADHESION AND ADHESIVE INTESTINAL OBSTRUCTION AFTER ABDOMINAL OPERATIONS IN RATS, Acta Acadeniae Medicinae Wuhan, 3, (2), pp. 77-83.

(The publication dates of the Ascherl et al. and Minju et al. articles are not known. The translations have the dates "7/18/85" printed on the title pages. It is possible that these two publications are not prior art to applicants.)

t-PA is known to possess a fibrinolytic activity. See for instance EP-A-0 041 449 and EP-A-0 174 835.

Human fibrinolysin has been evaluated, either alone or combined with other medicaments, to combat post-surgical adhesions. See Gazzaniga et al., Arch. Surg. Vol. 110, pages 429-432 (1975), and references cited therein.

Other authors have reported that fibrinolytic agents have either not been successful in preventing adhesions, or the risks associated with their use were too high. In this connection, see, for instance, the following articles:

Holtz, "Prevention of Postoperative Adhesions", The Journal of Reproductive Medicine, Vol. 24, No. 4, April 1980, pages 141-146, esp. page 144; and

Rivkind et al., Eur. Surg. Res. (Switzerland), 1985, Vol. 17, No. 4, pages 254-8.

Buckman et al., in "A Unifying Pathogenetic Mechanism in the Etiology of Intraperitoneal Adhesions", Journal of Surgical Research, Vol. 20, No. 1, January 1976 (pages 1-5), theorized that post-surgical adhesion formation in the peritoneal cavity was associated with trauma or ischemia induced reduction in plasminogen activator activity.

The present invention concerns the use of tissue plasminogen activator (t-PA) in the production of a topically applicable medicament for topical use in an amount effective in inhibiting the formation of post-

2

surgical adhesions in mammals.

The pharmacologically active substance employed in this invention is tissue plasminogen activator. t-PA is a product that is normally present in the body, where it plays a role in the lysis of thrombi. There is some evidence that in its "storage mode" in the body, t-PA exists in the single stranded form. t-PA can also exist in a double stranded form. Both forms have a high affinity for fibrin, a component of thrombi. Upon being bound to fibrin, the single stranded form of t-PA appears to be converted to the double stranded form. Both the single stranded and double stranded forms of t-PA can be used in this invention. Natural t-PA is glycosolated and contains associated fatty acid moieties. Both the degree of glycosolation and the number of and specific nature of the associated fatty acid moieties are believed to vary normally, and can also vary with the t-PA employed in this invention.

t-PA can be isolated from human tissue, and more recently has been produced using recombinant DNA technology. See, for instance, Goeddel et al., UK Patent Application GB 2 119 804 A, published November 23, 1983, Gill, European Patent Application 0 174 835, published March 19, 1986, and Mori et al., European Patent Application 0 100 982, published February 22, 1984, for various methods of isolating t-PA from tissue or producing it by r-DNA techniques.

The medicament is applied topically to the site of surgical trauma in effective amounts for, e.g., at least one and up to about seven days after the operation, to thereby inhibit the formation of post-surgical adhesions. It is preferably administered immediately post-operatively before wound healing has begun. By the term "topically", is meant that the t-PA is administered non-systemically to the surface of the tissue (internal or, in some cases, external) to be treated, for local effect. The term "site of surgical trauma" is meant to include the site of tissue that has been injured in any way, and includes, for example, tissue sites that have undergone incision, excision, abrasion, contusion, manipulation, laceration, anastomosis, prosthetic surgery, curettage, orthopedic surgery, neurosurgery, cardiovascular surgery, or plastic or reconstructive surgery. "Site of surgical trauma" also includes tissue that is adjacent to the injured tissue.

The medicament is useful in any surgical procedure in which it is desired to inhibit the formation of post-surgical adhesions. It is thus broadly useful in all types of surgery in which adhesion formation can be a complication.

The medicament may be administered to the site#of surgical trauma by any convenient mode such as, for example, by lavage, by catheter, by coating directly on the site in a salve, ointment, gel, cream, aqueous surface active composition, emulsion, suspension, or foam, or by any other convenient mode. The site can be contacted directly, as by applying a salve, or in some cases the medicament can be introduced to a site near the site of trauma and natural migration of fluids will serve to carry the medicament to the desired site. Such natural migration of fluids can occur, for instance, intraperitoneally, in response to peristaltic contraction of the intestines.

The t-PA is ordinarily administered in a sterile formulation in a pharmaceutically acceptable carrier or vehicle such as phosphate buffered saline ("PBS"), isotonic saline, purified water, an organic carrier such as a lipid, for example, a phospholipid micelle or vesicle, dextran, polymers (especially p-dioxanone, lactide, and/or glycolide based absorbable polymers), which may be in the form of microcapsules or which may be incorporated in a salve- or ointment-like formulation, or in an aqueous solution of a surface active agent such as a polyoxyethylene-polyoxypropylene block copolymer or a sorbitan fatty acid ester-polyoxyethylene ether. Sterilization of the formulation may be accomplished in the usual ways, including aseptic preparation, filtration, exposure to gamma radiation, autoclaving, and the like. In one preferred aspect of the invention, the t-PA is contained in a controlled release carrier that is capable of releasing the active drug for a period of at least one and up to about seven days. The mode of administration of the t-PA is preferably continually over the critical wound healing period, such as will be the case when the drug is administered via a controlled release carrier or by a catheter. Such controlled release carriers include absorbable polymers, which may be in the form of microcapsules or in a salve or ointment-like formulation, and phospholipid compositions, especially phospholipid vesicles which are referred to as "liposomes."

Methods for incorporating drugs in phospholipid carriers are known in the art. For instance, one procedure for encapsulating a drug in a phospholipid vesicle is the following:

a lipid or mixture of lipids such as lecithin or other phospholipid, which may be mixed with cholesterol or other lipoid substance, is dissolved in an organic solvent such as diethyl ether; and

an aqueous phase containing the material to be encapsulated (in this case, t-PA) is added to the lipid solution, and the mixture is agitated as by exposing it to ultrasonic sound waves (sonicated). Preferably, the organic solvent is removed during sonication, as by use of heat or vacuum or both, although in some cases the solvent can be removed after the sonication. This procedure typically produces a unilamellar vesicle.

Another procedure for producing a phospholipid vesicle (in this case a multilamellar vesicle "MLV") containing a medicament is to form a film of dry lipid, as by evaporating the solvent from an organic solvent solution containing a lipid to form a film on the walls of the vessel containing the solution, and then stirring in the aqueous phase containing the t-PA to be encapsulated. (The evaporation can be done by spray drying or by vacuum evaporation, or by any other convenient method.) Free unencapsulated t-PA can be separated from MLV's by centrifugation at, e. g., 12,000 rpm.

Preferably, the vesicle containing the t-PA is dehydrated, as by freeze drying, after preparation, in order to insure long term storage stability. The aqueous vesicle suspension can be reconstituted just prior to use by adding sterile phosphate buffered saline, sterile water, or the like.

The use of multilamellar vesicles of comparatively large size (e. g., from about 1 to about 10 μm) appears to be preferable in order to increase the dwell time of the vesicle containing the t-PA in the peritoneal cavity (or other body cavity). It is also preferred to use a pure or synthetic phosphatidylcholine in which the fatty acid moieties in the phosphatidylcholine are derived from a single fatty acid, in preparing the vesicle instead of natural lecithin, which is ordinarily a mixture of compounds. The fatty acid moieties in the liposomes are usually derived from $C_{12}$ to $C_{24}$ fatty acids, and preferably from $C_{14}$ to $C_{20}$ unsaturated fatty acids.

The following United States patents describe the preparation, by various procedures, of phospholipid vesicles containing various medicaments:

Rahman No. 3,993,754

Lenk et al. No. 4,522,803

Baldeschwieler et at. No. 4,310,505

Mezei et al. No. 4,485,054

Gersonde et al. No. 4,452,747

Kelly No. 4,356,167

Papahadjopoulos et al. No. 4,241,046

Suzuki et al. No. 4,016,100

Sache et al. No. 4,239,754

MacDonald No. 4,532,089

See also Callahan et al., European Patent Application No. 0126580, published November 28, 1984, and Gregoriadis, "The Carrier Potential of Liposomes In Biology and Medicine", New England Journal of Medicine, Vol. 295, pp. 704-710 and pp. 765-770 (Sept. 23 and 30, 1976).

The foregoing describe general procedures which can be utilized for the incorporation of t-PA in liposomes.

Other procedures for containing drugs in phospholipids (micelles or liposomes) are described in Sears, U.S. Patent Nos. 4,426,330 and 4,145,410, and Sears et al., U.S. Patent No. 4,298,594.

It is not essential that the t-PA medicament used in the invention be encapsulated in an inside compartment of the carrier as will normally be the case when the carrier is a phospholipid vesicle. In some cases it is acceptable for the t-PA to be dissolved or otherwise distributed more or less evenly throughout the carrier.

The t-PA is administered to the site of surgical trauma in effective quantities over the critical wound healing period (which period varies from patient to patient and with the type of surgical trauma encountered, but is usually from about one to five days and in some cases up to seven days or more, post-operatively.) The examples below illustrate the order of magnitude of effective quantities of the drug and the period of time over which the drug is administered for effective results.

The following studies use rabbit models to illustrate the adhesion inhibition effectiveness of the topical administration of t-PA to the site of surgical trauma:

Standardized Surgical Injury

New Zealand white female rabbits (1.8-2.0 kg) underwent midline laparotomy using acelepromazine and ketamine anaesthesia. A 3 x 5 cm flap of parietal peritoneum (about 1 mm thick) was sharply dissected from the right lateral peritoneal sidewall. The serosal surface of the adjacent large bowel was abraided with a scalpel until punctate bleeding developed. This area between the excised parietal peritoneum and adjacent large bowel serosa was then used for evaluating the efficacy of t-PA for adhesion prevention. A second incision of parietal peritoneum covering the same total area (about 15 cm²) was performed in some of the rabbits 1.5-2.0 cm inferior to the initial test site along the right lateral peritoneal sidewall. Abrasion of the adjacent large bowel serosa was performed as described above for the treatment site. This second area was used as a non-treated control site to determine the effectiveness of the surgical procedure in producing adhesions, and the response to vehicle controls.

Alzet mini pumps (model 2ML1, 2 ml volume, pumping rate 10 microliters per hour, Alza, Palo Alto see U. S. Patent No. 3,995,631) containing phosphate buffered saline with varying doses of tissue plasminogen activator (Genentech, South San Francisco, CA - the Genentech t-PA employed was predominantly double stranded t-PA) were sewn into the right dorsal subcutaneous space with Vicryl sutures placed 3-5 mm from each end of the pump. The polyethylene catheter tip leading from the pump into the peritoneal cavity was placed 2-3 mm over the injury test site. The catheter was secured by two 3-0 Vicryl sutures placed outside the site of injury. A similar pump and catheter system containing only Ringer's lactate was implanted in the middle portion of the inferior (control) test site.

Seven days after the day of abrasion, the rabbits were sacrificed by pentobarbital overdose. The extent of adhesions was evaluated as follows:

1. No adhesions
2. Filmy adhesions (separable)
3. Mild adhesions (not separable - covering up to about 35% of the test area)
4. Moderate adhesions (not separable - covering about 35 to 60 % of the test area)
5. Severe adhesions (not separable - covering greater than about 60% of the test area)

The evaluation ratings set forth above are useful in the context of comparing the efficacy of various means for inhibiting the formation of adhesions. However, it should be mentioned that ultimately only a rating of "1" can be considered to be completely acceptable. Clinical complications can result from even mild adhesions, although such complications are considered to be more likely to occur with severe adhesions than with mild or moderate adhesions.

Example 1

In the first series of experiments, nine rabbits were used. Four were vehicle controls in which the pumps contained isotonic buffer alone, and the other five were used to evaluate the efficacy of t-PA to combat post-surgical adhesion as described above. The mini pump in each t-PA test rabbit contained two ml of isotonic buffer containing 5 mg per ml of essentially pure t-PA. The results of the experiments are shown below in Table I:

Table I

| Rabbit No. | t-PA or Control | Adhesion Evaluation |
|:---:|:---:|:---:|
| 1 | Control | 5 |
| 2 | " | 5 |
| 3 | " | 4 |
| 4 | " | 5 |
| 5 | t-PA | 1* |
| 6 | " | 1* |
| 7 | " | 1 |
| 8 | " | 1* |
| 9 | " | 1 |

*Three of the rabbits exhibited bleeding at the site of the trauma; two bled lightly and the other moderately to severely.

Example 2

In order to try to better define the threshold dosage rate in this experimental model, similar experiments were carried out with the mini pump, using different concentrations of t-PA per pump. Table II, below, sets forth the dosage rates and responses in this series of experiments:

Table II

| Rabbit No. | mg t-PA Per ml of Buffer | Adhesion Evaluation |
|---|---|---|
| 1 | 3 | 1 |
| 2 | 3 | 1 |
| 3 | 1.5 | 1 |
| 4 | 1.5 | 1 |
| 5 | 1.5 | 1 |
| 6 | 0.5 | 4 |
| 7 | 0.5 | 4 |
| 8 | 0.15 | 1 |
| 9 | 0.15 | 1 |
| 10 | 0.15 | 3 |
| 11 | 0.05 | 1 |
| 12 | 0.05 | 3 |
| 13 | 0.05 | 4 |
| 14 | 0.015 | 5 |
| 15 | 0.015 | 5 |
| 16 | 0.015 | 4 |

These results indicate that the threshold dosage (for this Genentech t-PA) at which significant beneficial anti-adhesion properties are obtained is between 0.015 and 0.05 mg t-PA/ml of buffer. (The results observed with rabbit Nos. 6 and 7 appear to be anomalous.) No bleeding was encountered with any of these rabbits.

Since no bleeding was encountered with any of the rabbits in the dosage rate studies, it appears that in this model, a safe upper limit of dosage that would not result in significant bleeding would be found in the dosage rate administered to Rabbit Nos. 1 and 2 (in which the concentration of t-PA was 3 mg/ml).

Example 3

In order to determine the time period over which the anti-adhesion agent is preferably administered in order to have a significant anti-adhesion effect, the following series of experiments were carried out:
The pumps contained 10 mg of t-PA (5 mg/ml), and the catheter delivering the treatment solution to the site of the surgical trauma was disconnected 2, 3, and 5 days post-operatively. The rabbits were sacrificed 7 days post-operatively, and evaluated as above. The results are displayed in Table III, below.

Table III

| Rabbit No. | Post-Op Day Catheter Disconnected | Evaluation |
|---|---|---|
| 1 | 2 | 1 |
| 2 | 2 | 1 |
| 3 | 2 | 1 |
| 4 | 3 | 1 |
| 5 | 3 | 1 |
| 6 | 3 | 1 |
| 7 | 5 | 1 |
| 8 | 5 | 4 |
| 9 | 5 | 1 |

Example 4

In this experiment, t-PA obtained from Damon Biotech, Needham Heights, MA. This t-PA was predominantly single stranded t-PA. The experiment was similar to that described above in Example 2, except that a vehicle control site was used in each rabbit, as explained above in the Standardized Surgical Injury section. The results were as follows:

Table IV

| | | Evaluation | |
|---|---|---|---|
| Rabbit No | mg t-PA per ml buffer | t-PA Site | Vehicle Control Site |
| 1 | 0.5 | 1 | 5 |
| 2 | " | 1 | 5 |
| 3 | " | 1 | 1 |
| 4 | " | 1 | 5 |
| 5 | " | 3 | 4 |
| 6 | 0.15 | 1 | 1 |
| 7 | " | 1 | 1 (Bleeding) |
| 8 | " | 1 | 1 |
| 9 | " | 3 | 1 |
| 10 | " | 1 | 1 |
| 11 | 0.05 | 1 | 3 |
| 12 | " | 1 | 3 |
| 13 | " | 1 | 1 |
| 14 | " | 1 | 4 |
| 15 | " | 1 | 3 |
| 16 | 0.015 | 1 | 1 |
| 17 | " | 3 | 4 |
| 18 | " | 1 | 1 |
| 19 | " | 1 | 5 |
| 20 | " | 1 | 5 |

The Damon Biotech t-PA appears to be more active, on a weight basis, than the Genentech t-PA.

Urokinase and streptokinase were both evaluated for anti-adhesion activity in the rabbit model described above, and both were found to have no anti-adhesion activity.

It is relevant to note that when the vehicle control site is in the same rabbit as the test site, migration of fluid in the peritoneal cavity can carry some of the medicament from the treatment site to the vehicle control site. Therefore, it is possible that some vehicle control sites could have received small amounts of t-PA owing to migration or circulation of fluid within the peritoneal cavity. However, if any of the untreated control sites did receive some of the active medicament by such fluid migration, it would have been significantly less than that received at the treatment site in the same rabbit. Therefore, differences in results between the treatment sites and the control sites in the same rabbit can confidently be interpreted as being caused by the adhesion inhibition effect of the t-PA.

An effective dose of a topically applied drug is normally expressed in terms of concentration of the drug in the carrier, coupled with the number of times per day the drug is applied. In the present invention, the effective dose will be dependent upon factors such as nature of specific t-PA used, nature of vehicle, nature of tissue to be treated, type of trauma, and mode of delivery (i.e., continuous delivery by catheter or a one-time administration in a vehicle such as a controlled release vehicle). Therefore, no hard and fast rule can be formulated that will apply in all cases, and experiments analogous to those reported in Examples 2 and 4 will have to be performed in order to precisely define the threshold dosage for specific vehicle systems, for specific modes of delivery, etc. It is well within the ability of the person skilled in the art to carry out the necessary experiments to determine threshold dosages, after having read this disclosure.

The t-PA active ingredient is administered to the site of surgical trauma topically. Such topical administration can be by spraying, lavage, dripping on the site, by catheter administration, or the like. The exact method of administration chosen is not critical, as long as an effective dose is administered over the critical wound healing period, which can be determined by a series of experiments analogous to that described above in Example 3.

Referring to the question of the effective dose of t-PA in accordance with this invention, while no hard and fast numbers can be presented that will be applicable to all cases, the examples presented above can be referred to as a guide to determine the order of magnitude of t-PA to employ. Thus, a concentration of t-PA in the vehicle of at least about 0.015 milligrams of t-PA per milliliter of total vehicle or carrier can be expected to exhibit anti-adhesion activity in most cases.

## Claims

1. Use of t-PA in the production of a topically applicable medicament for topical use in an amount effective in inhibiting the formation of post-surgical adhesions in mammals.

2. The use according to claim 1 wherein the medicament includes a controlled release carrier which releases the active ingredient in an effective amount over a period of time.

3. The use according to claim 2 wherein said period of time is from about one to seven days.

4. The use according to either of claims 2 and 3 wherein said carrier is a phospholipid.

5. The use according to claim 4 wherein said phospholipid is a phospholipid vesicle.

6. The use according to either of claims 2 and 3 wherein said carrier is an absorbable polymer.

7. The use according to claim 1 wherein the medicament includes a carrier which is an aqueous composition including a surface active agent.

8. The use according to claim 1, wherein the medicament includes a carrier which is an aqueous isotonic buffer solution.

9. The use according to any one of claims 1 to 8 wherein the concentration of active ingredient in said medicament is about 0.015 milligrams per milliliter.

## Revendications

1. Utilisation de t-PA dans la production d'un médicament applicable en topique pour une utilisation topique, en une quantité efficace pour inhiber la formation d'adhérences post-chirurgicales chez les mammifères.

2. Utilisation selon la revendication 1, dans laquelle le médicament renferme un véhicule à libération prolongée qui libère l'ingrédient actif en une quantité efficace sur une période de temps.

3. Utilisation selon la revendication 2, dans laquelle ladite période de temps est d'environ un à sept jours.

4. Utilisation selon l'une quelconque des revendications 2 et 3, dans laquelle ledit véhicule est un phospholipide.

5. Utilisation selon la revendication 4, dans laquelle ledit phospholipide est une vésicule de phospholipide.

6. Utilisation selon l'une quelconque des revendications 2 et 3, dans laquelle ledit véhicule est un polymère absorbable.

7. Utilisation selon la revendication 1, dans laquelle le médicament renferme un véhicule qui est une composition aqueuse renfermant un agent tensioactif.

8. Utilisation selon la revendication 1, dans laquelle le médicament renferme un véhicule qui est une solution tampon aqueuse isotonique.

9. Utilisation selon l'une quelconque des revendications 1 à 8, dans laquelle la concentration en ingrédient actif dudit médicament est d'environ 0,015 milligramme par millilitre.

**Patentansprüche**

1. Verwendung von t-PA zur Herstellung eines topisch anwendbaren Medikaments zur topischen Verwendung in einer Menge, die bei der Hemmung der Bildung von postoperativen Adhäsionen bei Säugetieren wirksam ist.

2. Verwendung nach Anspruch 1, in der das Medikament einen Träger mit kontrollierter Freigabe enthält, der den Wirkstoff in einer wirksamen Menge über eine gewisse Zeit freisetzt.

3. Verwendung nach Anspruch 2, in der die Zeit von einem bis sieben Tagen beträgt.

4. Verwendung nach Anspruch 2 oder 3, in der der genannte Träger ein Phospholipid ist.

5. Verwendung nach Anspruch 4, in der das genannte Phospholipid ein Phospholipid-Bläschen ist.

6. Verwendung nach Anspruch 2 oder 3, in der der genannte Träger ein absorbierbares Polymer ist.

7. Verwendung nach Anspruch 1, in der das Medikament einen Träger enthält, der eine wäßrige, ein oberflächenaktives Mittel enthaltende Zusammensetzung ist.

8. Verwendung nach Anspruch 1, in der das Medikament einen Träger enthält, der eine wäßrige isotonische Pufferlösung ist.

9. Verwendung nach einem der Ansprüche 1 bis 8, in der die Konzentration des Wirkstoffs in dem genannten Medikament etwa 0,015 mg je ml ist.